# EUROPEAN PATENT APPLICATION

(11) **EP 1 391 212 A1**
(43) Date of publication of application: **25.02.2004**
(21) Application number: 02724622.2
(22) Date of filing: 19.04.2002
(51) Int. Cl.: A61K 47/32, A61K 47/38, A61K 9/00, A61J 3/06

(54) **ORAL PREPARATIONS AND SUPPORTS FOR ORAL PREPARATIONS**

(30) Priority: 24.04.2001 JP 2001125804
(71) Applicant: Lintec Corporation, Tokyo 173-0001 (JP)
(72) Inventor: NOGAMI, Eiji, Saitama-shi, Saitama 336-0026 (JP)
(74) Representative: Cresswell, Thomas Anthony
(86) International application number: PCT/JP2002/003920
(87) International publication number: WO 2002/087622

(57) **Abstract**

With an object of providing an orally administered agent (in particular a film-shaped orally administered agent) with which the ease and safety of taking the agent are improved, to attain this object, in an orally administered agent 1b having one drug-containing layer 11 and two water-swellable gel-forming layers 12, the water-swellable gel-forming layers 12 are provided, either directly or via intermediate layers, on the both faces of the drug-containing layer 11.

## Description

### TECHNICAL FIELD

The present invention relates to an orally administered agent and an orally administered agent/supporting substrate complex.

### BACKGROUND ART

There are cases in which compliance of taking an orally administered agent drops by an unpleasant feeling due to the drug being bitter, astringent or the like, nausea or vomiting due to taking the drug, or refusal to take the drug. Various forms of orally administered agents have thus been developed.

Typically used forms of orally administered agents are solid preparations such as tablets and capsules. However, these solid preparations are difficult to swallow as it is, and must generally be taken with a large amount of water; even when taken with a large amount of water, there are cases in which swallowing is still difficult. There are thus cases in which compliance of taking the drug drops. Moreover, there are cases in which a solid preparation mistakenly gets stuck in the trachea, and cases in which a solid preparation sticks to the esophagus and hence an esophageal ulcer forms in this place.

In particular, it may not be possible for elderly people and infants to swallow a solid preparation, andhence a drop in compliance of taking the drug is often seen. Moreover, in the case of bedridden patients, after putting the solid preparation into the patient's mouth, giving water slowly, and then waiting a while, it is necessary for the care provider to feel around with his/her own fingers inside the patient' s mouth to check that the solid preparation is not still there, and thus the work of checking whether or not the preparation has been taken is very burdensome.

To improve upon the difficulty of swallowing a solid preparation, and thus increase the ease and safety of taking the solid preparation, one can envisage making the form into a semi-solid form such as a jelly. However, a semi-solid preparation such as a jelly is difficult to realize due to the problems that a lot of moisture is contained, and hence the stability of the drug drops (particularly in the case of a readily hydrolyzable drug) , sterile handling is difficult during manufacture and storage, and packaging is expensive.

On the other hand, by processing an orally administered agent into a film-shaped preparation (sheet-shaped preparation), the moisture content in the preparation can be kept down, and hence the stability of the drug can be improved (particularly in the case of a readily hydrolyzable drug), handling becomes easy, and the packaging cost can be reduced.

Regarding such film-shaped preparations, film-shaped preparations for which an object is to make disintegration or dissolution occur rapidly in the mouth (Japanese Patent Application Laid-open No. H7-100186, Japanese Patent Application Laid-open No. H5-220203, Japanese Patent Application Laid-open No. H11-116469), and film-shaped preparations for which an object is to make handling of a minute amount of a drug easy (Japanese Patent Application Laid-open No. H5-124954) are known.

### DISCLOSURE OF THE INVENTION

However, these film-shaped preparations are not improved sufficientlywith regard to ease and safety of taking the preparation, strength of the film-shaped preparation, and so on.

For example, in the case of the above-mentioned film-shaped preparations for which an object is to make disintegration or dissolution occur rapidly in the mouth, the drug rapidly spreads through the mouth before the preparation is swallowed, and hence there is a drop in compliance of taking the drug due to the taste (e.g. bitterness, astringency) or smell of the drug. In the case of using a drug that is bitter or the like, processing such as making into a microcapsule is thus necessary.

Moreover, in the case of the above-mentioned film-shaped preparations for which an object is to make disintegration or dissolution occur rapidly in the mouth, it is not possible to visually check that a bedridden patient has taken the preparation, and hence as with a conventional solid preparation, this must be done by feeling around inside the patient's mouth, and thus the work of checking that the preparation has been taken is very burdensome.

Moreover, in the case of most of the above-mentioned film-shaped preparations, the film-shaped preparation is manufactured by mixing the drug and a component necessary for the film formation (e.g. a film-forming agent) together, and hence if the content of the drug in the film-shaped preparation increases, then the content of the component necessary for the film formation drops correspondingly, and hence the strength of the film-shaped preparation drops.

Moreover, in the case of the film-shaped preparations for which an object is to make handling of a minute amount of a drug easy, there are limitations on the types of drugs that can be contained in the preparation.

It is thus a first object of the present invention to provide an orally administered agent (in particular a film-shaped orally administered agent) according to which the ease and safety of taking the agent are improved.

Moreover, it is a second object of the present invention to provide a film-shaped orally administered agent that can contain a broad range of types of drugs.

Furthermore, it is a third object of the present invention to provide an orally administered agent (in particular a film-shaped orally administered agent) according to which a drop in compliance of taking a drug due to the taste (e.g. bitterness, astringency) or smell of the drug can be prevented.

Furthermore, it is a fourth object of the present invention to provide an orally administered agent/supporting substrate complex according to which handling of an orally administered agent (e.g. carrying, storage etc. of the orally administered agent) can be made easy.

Furthermore, it is a fifth object of the present invention to provide an orally administered agent/supporting substrate complex according to which an orally administered agent can be administered easily.

Furthermore, it is a sixth object of the present invention to provide an orally administered agent/supporting substrate complex according to which it can easily be checked whether or not an orally administered agent has been taken.

To attain the above objects, the present invention provides the following orally administered agent and orally administered agent/supporting substrate complex.
(1) An orally administered agent comprising a drug-containing layer and a water-swellable gel-forming layer, wherein the water-swellable gel-forming layer is provided as an outermost layer of the orally administered agent.
(2) The orally administered agent according to (1) above, wherein the orally administered agent is a film-shaped preparation.
(3) The orally administered agent according to (2) above, wherein the water-swellable gel-forming layer contains a water-swellable gel-forming agent and a film-forming agent.
(4) The orally administered agent according to (3) above, wherein the water-swellable gel-forming agent is a cross-linked carboxyvinyl polymer, and the film-forming agent is a polyvinyl alcohol.
(5) The orally administered agent according to (4) above, wherein the cross-linked carboxyvinyl polymer is a carboxyvinyl polymer cross-linked by a polyvalent metal compound.
(6) The orally administered agent according to any of (3) through (5) above, wherein the content of the water-swellable gel-forming agent in the water-swellable gel-forming layer is 15 to 70wt%, and the content of the film-forming agent in the water-swellable gel-forming layer is 30 to 85wt%.
(7) The orally administered agent according to any of (1) through (6) above, wherein the water-swellable gel-forming layer can mask the taste and/or smell of a drug contained in the drug-containing layer.
(8) The orally administered agent according to any of (1) through (7) above, wherein the drug-containing layer contains an edible polymer as a base.
(9) The orally administered agent according to (8) above, wherein the edible polymer is cellulose and/or a cellulose derivative.
(10) The orally administered agent according to (8) or (9) above, wherein the content of the edible polymer in the drug-containing layer is at least 20wt%.
(11) An orally administered agent/supporting substrate complex comprising the orally administered agent according to any of (1) through (10) above, and a supporting substrate that supports the orally administered agent, wherein the orally administered agent is provided on the supporting substrate either directly or via an intermediate layer.
(12) The orally administered agent/supporting substrate complex according to (11) above, wherein the supporting substrate has a gripping part and a mouth-inserting part, and the orally administered agent is provided on the mouth-inserting part.

### BRIEF DESCRIPTION OF THE DRAWINGS

Fig. 1 is a sectional view showing an embodiment of the orally administered agent of the present invention for the case that one drug-containing layer is provided;
Fig. 2 is a sectional view showing another embodiment of the orally administered agent of the present invention for the case that one drug-containing layer is provided;
Fig. 3 is a sectional view showing another embodiment of the orally administered agent of the present invention for the case that one drug-containing layer is provided;
Fig. 4 is a sectional view showing an embodiment of the orally administered agent of the present invention for the case that two drug-containing layers are provided;
Fig. 5 is a sectional view showing an embodiment of the orally administered agent of the present invention for the case that three drug-containing layers are provided;
Fig. 6 is a sectional view showing the state of the orally administered agent shown in Fig. 1 during administration;
Fig. 7 (a) is a top view showing an embodiment of the orally administered agent/supporting substrate complex of the present invention, and Fig. 7 (b) is a sectional view showing this embodiment;
Fig. 8(a) is a top view showing another embodiment of the orally administered agent/supporting substrate complex of the present invention, and Fig. 8 (b) is a sectional view showing this embodiment;
Fig. 9(a) is a top view showing yet another embodiment of the orally administered agent/supporting substrate complex of the present invention, and Fig. 9(b) is a sectional view showing this embodiment;
Fig. 10 (a) is a top view showing yet another embodiment of the orally administered agent/supporting substrate complex of the present invention, and Fig. 10 (b) is a sectional view showing this embodiment; and
Fig. 11 is a drawing showing an example of a method of manufacturing the orally administered agent of the present invention.

### BEST MODE FOR CARRYING OUT THE INVENTION

Following is a detailed description of the present invention.

The orally administered agent of the present invention has drug-containing layer(s) and water-swellable gel-forming layer(s). The orally administered agent of the present invention may have layer(s) other than the drug-containing layer(s) and the water-swellable gel-forming layer(s), or may be constituted from only the drug-containing layer(s) and the water-swellable gel-forming layer(s).

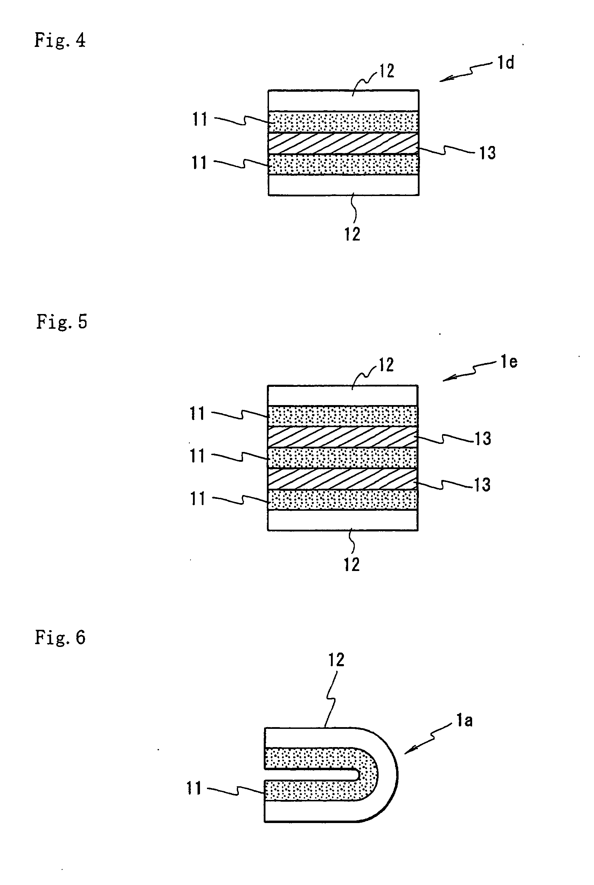

The orally administered agent of the present invention is a layered medicinal agent constituted from a plurality of layers built up on one another; however, the orally administered agent is not limited to having a flat shape as with a film-shaped preparation (sheet-shaped preparation), but rather so long as the orally administered agent is layered, the orally administered agent may have any shape. For example, the shape may be a shape formed by folding up a flat shape (see Fig. 6).

The orally administered agent of the present invention is preferably a film-shaped preparation. By processing into a film-shaped preparation, the moisture content in the preparation can be kept low, and hence the stability of the drug contained in the preparation can be increased (particularly in the case of a readily hydrolyzable drug). Moreover, handling of the preparation becomes easy, and the packaging cost can be reduced.

In the case that the orally administered agent of the present invention is film-shaped, layer(s) for adjusting the film thickness may be provided as layer(s) other than the drug-containing layer(s) and the water-swellable gel-forming layer(s). By providing such layer(s) and thus increasing the film thickness, the ease of handling of the orally administered agent of the present invention can be improved.

In the orally administered agent of the present invention, 'drug-containing layer' means a layer containing the drug to be administered. The thickness of the drug-containing layer(s) can be adjusted as appropriate in accordance with the drug content and so on within a thickness range for which oral administration can be carried out; in the case of making into a film-shaped preparation, the thickness of the drug-containing layer(s) is preferably 0.1 to 1000µm, more preferably 10 to 200µm. This is because if the thickness of the drug-containing layer(s) is less than 0.1µm, then it will be difficult to make the film with good precision (i.e. variation will arise in the drug content in the drug-containing layer(s)); on the other hand, if the thickness of the drug-containing layer(s) exceeds 1000µm, then the film will become stiff and hence taking the agent will become difficult.

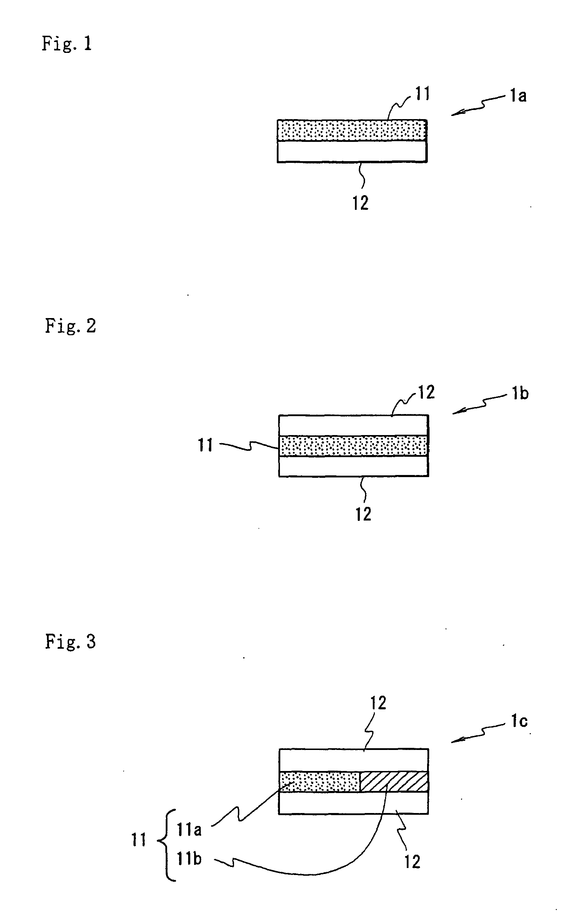

A single drug-containing layer may be provided in the orally administered agent of the present invention, or a plurality of drug-containing layers may be provided. In the case of providing a plurality of drug-containing layers in the orally administered agent of the present invention, the drug-containing layers may be laminated together directly, or may be laminated together via intermediate layer(s). Moreover, a single drug-containing layer may be formed from a plurality of drug-containing layers formed side by side (see Fig. 3).

The drug-containing layer(s) may comprise only the drug to be administered, but usually contain additive(s) such as pharmacologically acceptable excipient(s), binder(s) and disintegrator(s) as a base for keeping the drug to be administered in a desired state in the drug-containing layer. Moreover, masking agent(s), colorant(s) and so on may be included in the drug-containing layer(s) as described later.

There are no particular limitations on a base contained along with the drug in the drug-containing layer(s); a base can be selected as appropriate in accordance with the object of adding the base. Specific examples of bases that may be contained in the drug-containing layer(s) include cellulose and derivatives thereof, for example crystalline cellulose, carboxymethyl cellulose, hydroxypropyl cellulose, hydroxypropyl methyl cellulose, methyl cellulose, ethyl cellulose, cellulose acetate, cellulose acetate phthalate, hydroxypropyl methyl cellulose phthalate, hydroxypropyl methyl cellulose acetate succinate, and carboxymethyl ethyl cellulose, or pharmacologically acceptable salts thereof (e.g. a sodium salt); starches and derivatives thereof, for example α-starch, oxidized starch, sodium carboxymethyl starch, hydroxypropyl starch, dextrin, and dextran; saccharides, for example saccharose, maltose, lactose, glucose, fructose, pullulan, xanthan gum, and cyclodextrin; sugar alcohols, for example xylitol, mannitol, and sorbitol; acrylic acid derivatives, for example a dimethylaminoethyl methacrylate - methacrylic acid copolymer, a methacrylic acid - ethyl acrylate copolymer, a methacrylic acid - methyl methacrylate copolymer, an ethyl methacrylate-trimethylammonium methacrylate chloride copolymer, a dimethylaminoethyl methacrylate - methacrylate methyl chloride copolymer, and a methacrylic acid - acrylate ethyl chloride copolymer; shellac; polyvinyl acetal diethylamino acetate; vinyl acetate; polyvinyl alcohol; polyvinyl pyrrolidone; natural rubber, for example gum arabic, and tragacanth gum; polyglucosamines, for example chitin, and chitosan; proteins, for example gelatin, casein, and soy protein; titanium oxide; calcium monohydrogen phosphate; calcium carbonate; talc; stearates; magnesium aluminate metasilicate; magnesium silicate; and silicic anhydride; these can be used singly or two or more can be used in combination.

It is preferable for a base contained in the drug-containing layer(s) to be an edible polymer. The edible polymer may be a synthetic polymer or a natural polymer, and there are no particular limitations on the type thereof.

The edible polymer is preferably a stomach-soluble polymer or an intestine-soluble polymer.

Out of edible polymers, examples of preferable ones are cellulose and/or cellulose derivatives, and examples of particularly preferable ones include hydroxypropyl cellulose, and hydroxypropyl methyl cellulose phthalate. Hydroxypropyl cellulose and hydroxypropyl methyl cellulose phthalate have excellent film-forming properties, and hence are particularly useful in the case of making the drug-containing layer(s) film-shaped. By making the drug-containing layer(s) film-shaped, it becomes possible to make the orally administered agent of the present invention as a whole film-shaped.

The content of the edible polymer in each drug-containing layer is an amount such that formation of the layer is possible; this amount can be adjusted as appropriate in accordance with the type of the edible polymer and so on, but is usually at least 20wt%, preferably at least 60wt%, more preferably at least 70wt%, of the drug-containing layer. If the edible polymer content is less than 20wt%, then the formation of the drug-containing layer will be inadequate. Note that the upper limit of the content of the edible polymer in the drug-containing layer is the value obtained by subtracting the minimum content of the drug in the drug-containing layer from 100wt%, and is set as appropriate in accordance with the type of the drug and so on.

The drug contained in each drug-containing layer is a drug that is to be administered to the patient or the like, and so long as it is a drug that can be orally administered, there are no particular limitations thereon. Examples of drugs that can be orally administered include: as drugs that act on the central nervous system: hypnotics such as amobarbital, estazolam, triazolam, nitrazepam, and pentobarbital; psychotropic agents such as amitriptyline hydrochloride, imipramine hydrochloride, oxazolam, chlordiazepoxide, chlorpromazine, diazepam, sulpiride, and haloperidol; antiparkinsonism agents such as trihexyphenidyl , and levodopa; analgesics and antiphlogistics such as aspirin, isopropyl antipyrine, indomethacin, diclofenac sodium, mefenamic acid, streptokinase, streptodornase, serrapeptase, and pronase; and central nervous metabolism activators such as ATP, and vinpocetine; as drugs that act on the respiratory organs: expectorants such as carbocysteine, and bromhexine hydrochloride; and antiasthmatic agents such as azelastine hydrochloride, oxatomide, theophylline, terbutaline sulfate, tranilast, procaterol hydrochloride, and ketotifen fumarate; as drugs that act on the circulatory system: cardiotonics such as aminophylline, digitoxin, and digoxin; antiarrhythmic agents such as ajmaline, disopyramide, procainamide hydrochloride, and mexiletine hydrochloride; antianginal agents such as amyl nitrite, alprenolol hydrochloride, isosorbide nitrate, nicorandil, oxyfedrine, dipyridamole, dilazep hydrochloride, diltiazem hydrochloride, nitroglycerine, nifedipine, and verapamil hydrochloride; peripheral vasodilators such as kallidinogenase; antihypertensive agents such as atenolol, captopril, clonidine hydrochloride, metoprolol tartrate, spironolactone, triamterene, trichlormethazide, nicardipine, hydralazine hydrochloride, hydrochlorothiazide, prazosin hydrochloride, furosemide, propranolol hydrochloride, enalapril maleate, methyldopa, labetalol hydrochloride, and reserpine; and antiarteriosclerotic agents such as clofibrate, dextran sulfate, nicomol, and niceritrol; as drugs that act on the blood or have a hemopoietic action: hemostatics such as carbazochrome sodium sulfonate, and tranexamic acid; antithrombotic agents such as ticlopidine hydrochloride, and warfarin potassium; and therapeutic drugs for anemia such as iron sulfate; as drugs that act on the digestive system: antiulcer agents such as azulene, aldioxa, cimetidine, ranitidine hydrochloride, famotidine, teprenone, and rebamipide; antiemetics such as domperidone, and metoclopramide; evacuants such as sennoside; digestive enzyme preparations; and therapeutic drugs for liver disorders such as glycyrrhizin, and liver extract preparations; as drugs that act on metabolic disorders: antidiabetic agents such as glibenclamide, chlorpropamide, and tolbutamide; therapeutic drugs for gout such as allopurinol, and colchicine; as a drug in the ophthalmological field: acetazolamide; as drugs in the otorhinological field: anti-vertigo drugs such as difenidol hydrochloride, andbetahistine mesilate; as chemotherapeutic agents and antibiotics: isoniazid, ethambutol hydrochloride, ofloxacin, erythromycin stearate, cefaclor, norfloxacin, fosfomycin calcium, minocycline hydrochloride, rifampicin, rokitamycin, etc.; as drugs against malignant tumors: cyclophosphamide, tegafur etc.; as immunosuppressants: azathioprine, etc.; as hormones and endocrine therapeutic drugs: progesterone, salivary gland hormone, thiamazole, prednisolone, betamethasone, liothyronine, levothyroxine, etc.; and as autacoids: antihistamines such as clemastinefumarate, and d-chlorpheniramine maleate; and vitamins such as alfacalcidol, cobamamide, tocopherol nicotinate, and mecobalamin.

There are no particular limitations on the content of the drug in each drug-containing layer; this content can be adjusted as appropriate in accordance with the type of the drug, but is usually not more than 80wt%, preferably not more than 40wt%, more preferably not more than 30wt%, of the drug-containing layer. If the content of the drug exceeds 80wt%, then the film strength of the film-shaped preparation will drop. Note that the lower limit of the drug content in the drug-containing layer is set as appropriate in accordance with the type of the drug in the drug-containing layer, and is usually about 0.001wt%.

A broad range of types of drugs from drugs for which the dose is minute to drugs for which the dose is large can be contained in the drug-containing layer(s). Here, the dose being minute means that a single dose is 1mg or less, and the dose being large means that a single dose is 300mg or more.

Even in the case that the orally administered agent of the present invention is a film-shaped preparation, a broad range of types of drugs from drugs for which the dose is minute to drugs for which the dose is large, or insoluble bulky drugs that are prone to bringing about a drop in the film strength, can be contained in the drug-containing layer(s). This isbecause the drug-containing layer(s) and the water-swellable gel-forming layer(s) are formed as separate layers, and hence even if the drug content in the drug-containing layer(s) increases and thus the film strength of the drug-containing layer(s) drops, the strength of the film-shaped preparation as a whole can still be maintained by giving the water-swellable gel-forming layer(s) film-forming properties. The film-shaped orally administered agent of the present invention is thus particularly useful with the administration of a drug for which the dose is large or a bulky drug.

In the orally administered agent of the present invention, 'water-swellable gel-forming layer' means a layer that contains a water-swellable gel-forming agent, and is thus able to swell through moisture to form a gel. The thickness of the water-swellable gel-forming layer(s) can be adjusted as appropriate within a thickness range for which oral administration can be carried out; in the case of making into a film-shaped preparation, the thickness of the water-swellable gel-forming layer(s) is preferably 10 to 1000µm, more preferably 20 to 500µm. If the thickness of the water-swellable gel-forming layer(s) is less than 10µm, then gel formation will be inadequate, and masking of the taste and/or smell of the drug by the water-swellable gel-forming layer(s) will be inadequate; on the other hand, if the thickness of the water-swellable gel-forming layer (s) exceeds 1000µm, then it will not be possible for swelling and gel formation to take place sufficiently just through saliva upon administration into the mouth of a patient or the like, and hence taking the agent will become difficult.

There are no particular limitations on the type of the water-swellable gel-forming agent providing that it is capable of swelling through moisture to form a gel; the water-swellable gel-forming agent may be a cross-linked one or a non-cross-linked one. Specific examples of the water-swellable gel-forming agent include carboxyvinyl polymers, starches and derivatives thereof, agar, alginic acid, arabinogalactan, galactomannan, cellulose and derivatives thereof, carrageen, dextran, tragacanth, gelatin, pectin, hyaluronic acid, gellan gum, collagen, casein, and xanthan gum.

Out of these water-swellable gel-forming agents, a cross-linked carboxyvinyl polymer is preferable, with cross-linked polyacrylic acid being particularly preferable. A cross-linked carboxyvinyl polymer, and in particular cross-linked polyacrylic acid, does not exert adverse effects on the film-forming ability of the film-forming agent, and is capable of exhibiting a suitable gel strength upon swelling.

Cross-linking can be carried out using a cross-linking agent in accordance with the type of molecule to be cross-linked. A carboxyvinyl polymer can be cross-linked using, for example, a polyvalent metal compound. Specific examples of such a polyvalent metal compound include calcium chloride, magnesium chloride, aluminum chloride, aluminum sulfate, aluminum potassium sulfate, iron chloride alum, ammonium alum, ferric sulfate, aluminum hydroxide, aluminum silicate, aluminum phosphate, iron citrate, magnesium oxide, calcium oxide, zinc oxide, and zinc sulfate.

In the case of making the orally administered agent of the present invention into a film-shaped preparation, the water-swellable gel-forming layer(s) must be made film-shaped; in this case, to improve the film-forming properties of the water-swellable gel-forming layer(s), it is preferable to include a film-forming agent in the water-swellable gel-forming layer(s).

So long as the film-forming agent has a film-forming ability, there are no particular limitations on the type thereof. Specific examples of the film-forming agent include polyvinyl alcohol, polyvinyl pyrrolidone, polyvinyl acetate, polyvinyl acetate phthalate, hydroxyalkyl celluloses (e.g. hydroxypropyl cellulose, hydroxypropyl methyl cellulose, hydroxymethyl cellulose, hydroxyethyl cellulose), alkyl celluloses (e.g. methyl cellulose, ethyl cellulose), carboxyalkyl celluloses (e.g. carboxymethyl cellulose), (meth)acrylic acid and esters thereof, xanthan gum, carrageenan, and alginic acid.

The film-forming agent is preferably water-soluble. This is because in the case that the film-forming agent is water-soluble, it becomes easy for moisture to infiltrate into the water-swellable gel-forming layer(s), and hence swelling of the water-swellable gel-forming layer(s) to form a gel can be made to occur rapidly in the mouth.

Examples of water-soluble film-forming agents include polyvinyl alcohol; hydroxyalkyl celluloses such as hydroxypropyl cellulose, and hydroxypropyl methyl cellulose; polyvinyl pyrrolidone; xanthan gum; carrageenan; and alginic acid.

To give the water-swellable gel-forming layer(s) a suitable pliability, a plasticizer may be included in the water-swellable gel-forming layer(s). Examples of such a plasticizer include propylene glycol, polyethylene glycol, glycerin and sorbitol, glycerin triacetate, diethylphthalateand triethylcitrate, lauric acid, saccharose, and sorbitol. Note that such a plasticizer may also be included in the drug-containing layer(s), and in intermediate layer(s), mentioned later, as well as in the water-swellable gel-forming layer(s).

Moreover, a masking agent for masking the taste or smell of the drug in the drug-containing layer(s) may be included in the water-swellable gel-forming layer(s). By including a masking agent in the water-swellable gel-forming layer(s), the effect of the water-swellable gel-forming layer(s) masking the taste or smell of the drug can be improved, and hence a drop in compliance of taking the drug can be efficiently prevented.

As a masking agent, for example an agent giving an acid taste such as citric acid, tartaric acid or fumaric acid, a sweetening agent such as saccharin, glycyrrhizinic acid, saccharose, fructose or mannitol, a refrigerative such as menthol, mint oil, peppermint or spearmint, or a natural or synthetic flavoring can be used.

In the case that polyvinyl alcohol or the like is contained in the water-swellable gel-forming layer(s) as a film-forming agent, this film-forming agent will also be able to fulfil the role of a masking agent. In this way, it is preferable to use a film-forming agent having a masking action, and it is similarly preferable to use a water-swellable gel-forming agent having a masking action.

Moreover, preservatives such as methyl hydroxybenzonate and propyl hydroxybenzonate, colorants such as edible lake colorants, and so on may also be included in the water-swellable gel-forming layer(s).

In general the introduction of such additives causes a reduction in the strength of the film-shaped water-swellable gel-forming layer(s), and as a result it becomes easier for moisture to infiltrate into the water-swellable gel-forming layer(s), and hence it becomes easier for swelling of the water-swellable gel-forming agent and thus gel formation to take place through moisture that has infiltrated into the water-swellable gel-forming layer(s).

In the orally administered agent of the present invention, water-swellable gel-forming layer(s) is/are provided as outermost layer(s).

So long as water-swellable gel-forming layer(s) is/are provided as outermost layer(s) of the orally administered agent of the present invention, the water-swellable gel-forming layer(s) may be provided over the whole of the faces (or almost the whole of the faces) of the orally administered agent of the present invention, or may be provided on some of the faces of the orally administered agent; however, from the standpoint of efficiently exhibiting the effects of the water-swellable gel-forming layer(s) (improving the ease and safety of taking the agent, preventing a drop in compliance of taking the drug, etc.), it is preferable for the water-swellable gel-forming layer(s) to be provided over the whole of the faces (or almost the whole of the faces) of the orally administered agent of the present invention. For example, in the case that the orally administered agent of the present invention is a film-shaped preparation, out of the two outside faces of the film-shaped preparation, either one face or both faces can be made to be a water-swellable gel-forming layer, but it is preferable to make both faces a water-swellable gel-forming layer.

Here, 'outermost layer' means a layer that constitutes an outside face of the orally administered agent of the present invention when the orally administered agent of the present invention is inside the mouth of a patient or the like. 'Outermost layer' thus obviously includes a layer that constitutes an outside face of the orally administered agent of the present invention before administration, but also includes a layer that does not constitute an outside face of the orally administered agent of the present invention before administration but does constitute an outside face of the orally administered agent of the present invention when the orally administered agent is inside the mouth of a patient or the like. For example, even in the case that another layer is provided as a layer further to the outside than a water-swellable gel-forming layer, if this outer layer disintegrates or dissolves through moisture in saliva or the like inside the mouth of the patient or the like, then the water-swellable gel-forming layer comes to constitute an outside face of the orally administered agent of the present invention when the orally administered agent is inside the mouth of the patient, and hence this means that the water-swellable gel-forming layer is provided as an 'outermost layer' of the orally administered agent of the present invention.

So long as water-swellable gel-forming layer(s) constitute outside face(s) of the orally administered agent of the present invention when the orally administered agent of the present invention is inside the mouth of a patient or the like, only one water-swellable gel-forming layer may be provided in the orally administered agent of the present invention, or a plurality of water-swellable gel-forming layers may be provided.

In the case that one drug-containing layer is provided in the orally administered agent of the present invention, for example a water-swellable gel-forming layer can be provided either on one face or on both faces of the drug-containing layer, this being either directly or via an intermediate layer. One embodiment of the form of the agent for the case that one drug-containing layer is provided is shown in Fig. 1. As shown in Fig. 1, the orally administered agent 1a has one drug-containing layer 11 and one water-swellable gel-forming layer 12, with the water-swellable gel-forming layer 12 being provided directly on one face of the drug-containing layer 11.

Moreover, another embodiment for the case that one drug-containing layer is provided is shown in Fig. 2. As shown in Fig. 2, the orally administered agent 1b has one drug-containing layer 11 and two water-swellable gel-forming layers 12, with the water-swellable gel-forming layers 12 being provided directly on both faces of the drug-containing layer 11.

Moreover, another embodiment for the case that one drug-containing layer is provided is shown in Fig. 3. As shown in Fig. 3, the orally administered agent 1c has one drug-containing layer 11 that comprises a drug-containing layer 11a and a drug-containing layer 11b that are formed side by side, and two water-swellable gel-forming layers 12, with the water-swellable gel-forming layers 12 being provided directly on both faces of the drug-containing layer 11.

In the case that a plurality of drug-containing layers are provided in the orally administered agent of the present invention, for example water-swellable gel-forming layer(s) can each be provided, either directly or via an intermediate layer, on the outside face of a drug-containing layer positioned most to the outside (i. e. the face of this drug-containing layer on the opposite side to the face that is in contact with an intermediate layer or another drug-containing layer). One embodiment of the form of the agent for the case that a plurality of drug-containing layers are provided is shown in Fig. 4. As shown in Fig. 4, the orally administered agent 1d has two drug-containing layers 11, two water-swellable gel-forming layers 12, and one intermediate layer 13; the two drug-containing layers 11 are laminated together via the intermediate layer 13, and each water-swellable gel-forming layer 12 is directly provided on the outside face of one of the two drug-containing layers 11 (i.e. the face of this drug-containing layer 11 on the opposite side to the face that is in contact with the intermediate layer 13).

Moreover, another embodiment for the case that a plurality of drug-containing layers are provided is shown in Fig. 5. As shown in Fig. 5, the orally administered agent 1e has three drug-containing layers 11, two water-swellable gel-forming layers 12, and two intermediate layers 13; the three drug-containing layers 11 are laminated together via the intermediate layers 13, and each water-swellable gel-forming layer 12 is directly provided on the outside face of one of the three drug-containing layers 11 that is positioned most to the outside (i.e. the uppermost drug-containing layer or the lowermost drug-containing layer in Fig. 5) (i.e. the face of this drug-containing layer 11 on the opposite side to the face that is in contact with one of the intermediate layers 13).

In the orally administered agents 1a to 1e shown in Figs. 1 to 5, the water-swellable gel-forming layer(s) 12 may each be provided on the respective drug-containing layer 11 via an intermediate layer.

Intermediate layer(s) can be provided between the layers that constitute the orally administered agent of the present invention (e.g. between a drug-containing layer and a water-swellable gel-forming layer, between two drug-containing layers, between a water-swellable gel-forming layer or a drug-containing layer and a supporting substrate); the constituents of the intermediate layer(s) can be selected as appropriate in accordance with the purpose. For example, in the case of providing an intermediate layer with a purpose of bonding two layers together, a pharmacologically acceptable adhesive is included. Out of such adhesives, specific examples of adhesives that exhibit adhesiveness when used in a state containing a solvent include carboxyvinyl polymers, polyacrylic acid and pharmacologically acceptable non-toxic salts thereof such as sodium polyacrylate, acrylic acid copolymers and pharmacologically acceptable salts thereof, hydrophilic cellulose derivatives such as carboxymethyl cellulose and sodium salts, pullulan, povidone, karaya gum, pectin, xanthan gum, tragacanth, alginic acid, gum arabic, and acidic polysaccharides and derivatives or pharmacologically acceptable salts thereof; specific examples of adhesives that exhibit adhesiveness when heated (i.e. heat-sealing adhesives) include a homopolymer of vinyl acetate, and a copolymer between vinyl acetate and vinyl pyrrolidone.

In the orally administered agent of the present invention, because the water-swellable gel-forming layer(s) is/are provided as outermost layer(s), the water-swellable gel-forming layer(s) swell through moisture in saliva or the like in the mouth of a patient or the like to form a gel. As a result, the orally administered agent of the present invention changes into a form having a size, shape, elasticity, viscosity and so on such that swallowing is easy; the orally administered agent can thus be easily taken, and moreover the risk of the orally administered agent getting stuck in the trachea of the patient or the like is reduced, and thus the orally administered agent can be taken safely even by an elderly person or an infant. Moreover, in the case of a patient or the like who has little saliva and hence the water-swellable gel-forming layer(s) do not swell and form a gel sufficiently, the same effects can be produced by making the patient or the like take the orally administered agent with a little water, or by dipping the orally administered agent in water before administration. The amount of water required in this case is very small compared with the amount of water required when taking a solid preparation such as a tablet or a capsule.

Moreover, in the orally administered agent of the present invention, because the water-swellable gel-forming layer(s) is/are provided as outermost layer(s), the orally administered agent can be administered to a patient or the like in a state in which the whole (or almost the whole) of the outside faces of the orally administered agent is covered by the water-swellable gel-forming layer(s); as a result, the taste (e.g. bitterness, astringency) or smell of the drug in the drug-containing layer is masked, and hence a drop in compliance of taking the drug can be prevented. For example, in the case of the form of the agent shown in Fig. 1, by administering the orally administered agent 1a folded in two so that the whole (or almost the whole) of the outside faces of the orally administered agent 1a is covered by the water-swellable gel-forming layer 12 as shown in Fig. 6, the taste or smell of the drug in the drug-containing layer 11 can be masked. Moreover, in the cases of the forms of the agent shown in Figs. 2 to 5, because the whole (or almost the whole) of the outside faces of the orally administered agent 1b, 1c, 1d or 1e is coveredby the water-swellable gel-forming layers 12, if the orally administered agent is administered as it is, then the taste or smell of the drug in the drug-containing layer 11 can be masked.

The orally administered agent of the present invention can be supported on a supporting substrate. In an orally administered agent/supporting substrate complex of the present invention, the orally administered agent of the present invention is provided on the supporting substrate either directly or via an intermediate layer. By supporting the orally administered agent of the present invention on a supporting substrate in this way, handling of the orally administered agent of the present invention (e.g. carrying, storage etc. of the orally administered agent) becomes easy. Moreover, by forming the orally administered agent of the present invention on a supporting substrate, manufacture of the orally administered agent of the present invention becomes easy.

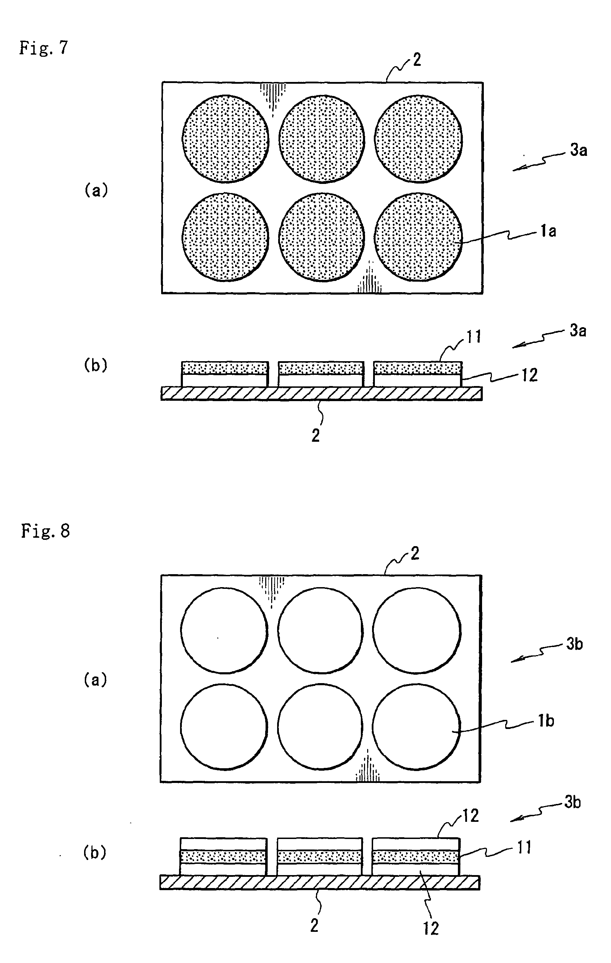

An embodiment of the orally administered agent/supporting substrate complex of the present invention is shown in Fig. 7. Note that Fig. 7(a) is a top view of the orally administered agent/supporting substrate complex according to this embodiment, and Fig. 7(b) is a sectional view of the orally administered agent/supporting substrate complex according to this embodiment.

The orally administered agent/supporting substrate complex 3a shown in Fig. 7 has, on a sheet-shaped supporting substrate 2, orally administered agents 1a each comprising a drug-containing layer 11 and a water-swellable gel-forming layer 12; the drug-containing layer 11 of each orally administered agent 1a is provided on one face of the sheet-shaped supporting substrate 2 via the water-swellable gel-forming layer 12. In the orally administered agent support 3a, orally administered agents 1a may also be provided on both faces of the sheet-shaped supporting substrate 2. Moreover, in the orally administered agent/supporting substrate complex 3a shown in Fig. 7, the number of orally administered agents 1a supported on the sheet-shaped supporting substrate 2 is six, but this number can be changed as appropriate.

Moreover, another embodiment of the orally administered agent/supporting substrate complex of the present invention is shown in Fig. 8. Note that Fig. 8 (a) is a top view of the orally administered agent/supporting substrate complex according to this embodiment, and Fig. 8(b) is a sectional view of the orally administered agent/supporting substrate complex according to this embodiment.

The orally administered agent/supporting substrate complex 3b shown in Fig. 8 has, on a sheet-shaped supporting substrate 2, orally administered agents 1b each constituted such that a drug-containing layer 11 is sandwiched between water-swellable gel-forming layers 12; the orally administered agents 1b are provided on one face of the sheet-shaped supporting substrate 2 via the water-swellable gel-forming layers 12. In the orally administered agent/supporting substrate complex 3b, orally administered agents 1b may also be provided on both faces of the sheet-shaped supporting substrate 2. Moreover, in the orally administered agent/supporting substrate complex 3b shown in Fig. 8, the number of orally administered agents 1b supported on the sheet-shaped supporting substrate 2 is six, but this number can be changed as appropriate.

In the orally administered agent/supporting substrate complex of the present invention, there are no particular limitations on the material of the supporting substrate, provided that this material can be molded; either an insoluble material that does not dissolve in the mouth of a patient or the like, or a soluble material that does dissolve in the mouth of a patient or the like may be used. As an insoluble material, for example a plastic such as polyethylene terephthalate, polyethylene, polypropylene or polyvinyl acetate, or paper or the like can be used. Moreover, as a soluble material, for example edible polymers such as polyvinyl alcohol, polyvinyl pyrrolidone, hydroxyalkyl celluloses (e.g. hydroxypropyl cellulose, hydroxypropyl methyl cellulose, hydroxymethyl cellulose, hydroxyethyl cellulose), alkyl celluloses (e.g. methyl cellulose, ethyl cellulose), carboxyalkyl celluloses (e.g. carboxymethyl cellulose), (meth) acrylic acid and esters thereof, carboxyvinyl polymers, starches and derivatives thereof, agar, alginic acid, arabinogalactan, galactomannan, cellulose and derivatives thereof, carrageen, dextran and tragacanth can each be used alone or two or more thereof can be used in combination.

There are no particular limitations on the shape of the supporting substrate, but the supporting substrate is preferably sheet-shaped, more preferably the sheet-shaped supporting substrate has a gripping part and a mouth-inserting part, with the orally administered agent of the present invention being provided on the mouth-inserting part.

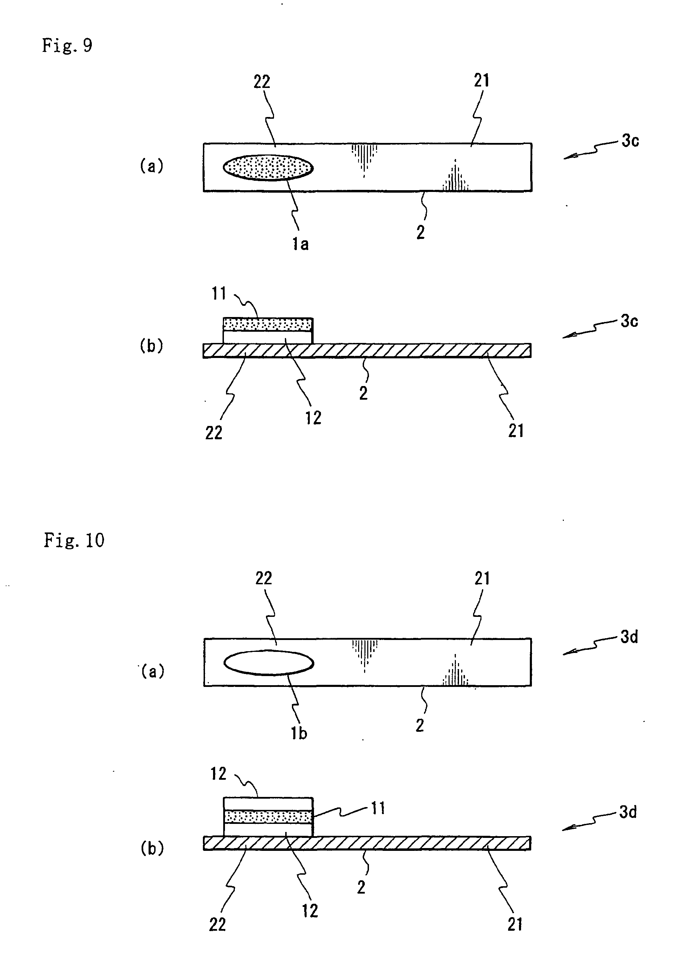

An embodiment of an orally administered agent/supporting substrate complex comprising a sheet-shaped supporting substrate having a gripping part and a mouth-inserting part is shown in Fig. 9. Note that Fig. 9(a) is a top view of the orally administered agent/supporting substrate complex according to this embodiment, and Fig. 9(b) is a sectional view of the orally administered agent/supporting substrate complex according to this embodiment.

The orally administered agent/supporting substrate complex 3c shown in Fig. 9 has a supporting substrate 2 having a gripping part 21 and a mouth-inserting part 22, and an orally administered agent 1a that is supported on the mouth-inserting part 22 of the supporting substrate 2.

Moreover, another embodiment of the orally administered agent/supporting substrate complex comprising a sheet-shaped supporting substrate having a gripping part and a mouth-inserting part is shown in Fig. 10. Note that Fig. 10(a) is a top view of the orally administered agent/supporting substrate complex according to this embodiment, and Fig. 10 (b) is a sectional view of the orally administered agent/supporting substrate complex according to this embodiment.

The orally administered agent/supporting substrate complex 3d shown in Fig. 10 has a supporting substrate 2 having a gripping part 21 and a mouth-inserting part 22, and an orally administered agent 1b that is supported on the mouth-inserting part 22 of the supporting substrate 2. The supporting substrate 2 of the orally administered agent/supporting substrate complexes 3c and 3d has a strip shape in which the gripping part 21 and the mouth-inserting part 22 are molded as a single body. In the orally administered agent/supporting substrate complexes 3c and 3d, the number of orally administered agents 1a or 1b supported on the supporting substrate 2 is one, but this number can be changed as appropriate.

In the supporting substrate, the gripping part and the mouth-inserting part may be formed as a single body, or may be formed separately to one another. There are no particular limitations on the shape, structure, size and so on of the gripping part provided that the gripping part can be grasped with the hand, and there are no particular limitations on the shape, structure, size and so on of the mouth-inserting part provided the mouth-inserting part can support the orally administered agent of the present invention and can be inserted into the mouth of a patient or the like.

According to the orally administered agent/supporting substrate complex comprising a supporting substrate having a gripping part and a mouth-inserting part in this way, by holding the gripping part of the supporting substrate with the hand, and inserting the mouth-inserting part on which the orally administered agent of the present invention is supported into the mouth of a patient or the like, the orally administered agent of the present invention can easily be administered. Moreover, by taking the mouth-inserting part out from the mouth of the patient or the like, and checking for the presence/absence of the orally administered agent of the present invention on the mouth-inserting part, it can easily be checked visually whether or not the agent has been taken.

In the case that the supporting substrate is constituted from a soluble material that dissolves in the mouth, by inserting the mouth-inserting part on which the orally administered agent of the present invention is supported into the mouth of the patient or the like, the mouth-inserting part dissolves in the mouth of the patient or the like and thus falls off, and hence the orally administered agent of the present invention can be administered in a shorter time than in the case that the supporting substrate is constituted from an insoluble material that does not dissolve in the mouth.

The orally administered agent and the orally administered agent/supporting substrate complex of the present invention can, for example, be manufactured as follows.

A suspension to which a water-swellable gel-forming agent and a film-forming agent have been added (with the solvent being, for example, purified water) is applied, sprayed or the like onto a supporting substrate such as a plastic film or a paper mounting, and then drying is carried out, thus forming a water-swellable gel-forming layer. A suspension to which a drug and additive(s) such as excipient(s) , binder(s) and disintegrator(s) have been added (with the solvent being, for example, ethanol) is then applied, sprayed or the like onto the upper surface of the water-swellable gel-forming layer that has been formed, and drying is carried out, thus forming a drug-containing layer. In the case of further forming a water-swellable gel-forming layer on the upper surface of the drug-containing layer, a suspension to which a water-swellable gel-forming agent and a film-forming agent have been added is applied on, sprayed on or the like, and then drying is carried out, as above. In this way, an orally administered agent/supporting substrate complex in which an orally administered agent is supported on the upper surface of a supporting substrate (e.g. an orally administered agent/supporting substrate complex as shown in any of Figs. 7 to 10) can be manufactured. Moreover, by peeling the supporting substrate off from the orally administered agent/supporting substrate complex, an orally administered agent (e.g. an orally administered agent as shown in any of Figs. 1 to 5) can be manufactured. If necessary, the orally administered agent/supporting substrate complex and the orally administered agent may be punched out into any desired shape such as a circle, an ellipse or a polygon, and slits may be inserted.

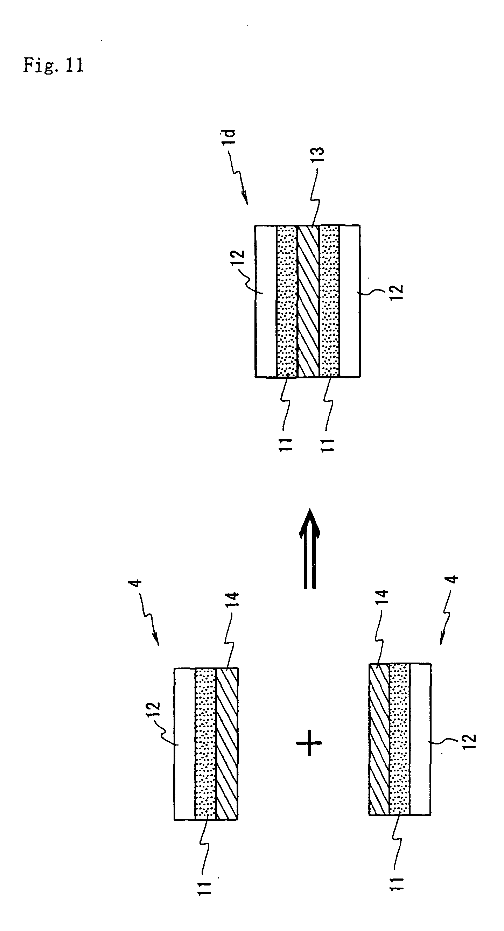

Moreover, after forming a water-swellable gel-forming layer on top of a supporting substrate such as a plastic film or a paper mounting and then forming a drug-containing layer on the upper surface of the water-swellable gel-forming layer as described above, an adhesive layer can be formed on the upper surface of the drug-containing layer by for example applying on or spraying on and then drying a solution containing an adhesive that exhibits adhesiveness upon heating. As a result, a medicinal agent in which a water-swellable gel-forming layer, a drug-containing layer and an adhesive layer have been built up in this order on the upper surface of a supporting substrate (the medicinal agent 4 shown in Fig. 11) can be manufactured. By, for example, thermally fusing together the adhesive layers 14 of medicinal agents 4 that have been manufactured in this way, an orally administered agent 1d in which two drug-containing layers 11 are laminated together via an intermediate layer 13 and water-swellable gel-forming layers 12 are provided as outermost layers (the orally administered agent 1d shown in Fig. 4) can be manufactured. In this case, by supporting one of the medicinal agents 4 that are to be thermally fused together on a supporting substrate in advance, an orally administered agent/supporting substrate complex in which an orally administered agent is supported on the upper surface of a supporting substrate can be manufactured.

Following is a more detailed description of the present invention through manufacturing examples and test examples.

### [Manufacturing examples 1]

### Manufacture of orally administered agents and orally administered agent/supporting substrate complexes

### (1) Manufacture of stomach-soluble preparation and stomach-soluble preparation/supporting substrate complex

A liquid A of the composition shown in Table 1 below was prepared for forming water-swellable gel-forming layers. That is, 45g of purified water was taken, and 10.5g of polyvinyl alcohol (Gohsenol EG05T (Nippon Synthetic Chemical Industry Co., Ltd.)) was added thereto slowly with stirring, and was then dissolved completely by stirring for about 1 hour while heating to 70°C. Similarly, 40g of purified water was taken, and 4.05g of polyacrylic acid (Junlon PW-111 (Nihon Junyaku Co., Ltd.)) was added thereto slowly with stirring, and was then dissolved completely by stirring for about 30 minutes. These two solutions were combined and thorough stirring was carried out, and then 0.45g of calcium chloride was added, and stirring was carried out for a further 5 minutes. Note that the polyacrylic acid is cross-linked by calcium ions that are generated through the calcium chloride undergoing electrolytic dissociation, and the cross-linked polyacrylic acid fulfils the role of a water-swellable gel-forming agent, while the polyvinyl alcohol fulfils the role of a film-forming agent.

**[Table 1]**

| Liquid A | |
|---|---|
| Polyvinyl alcohol | 10.5g |
| Polyacrylic acid | 4.05g |
| Calcium chloride | 0.45g |
| Purified water | 85.0g |

A liquid B of the composition shown in Table 2 below was prepared for forming a drug-containing layer. That is, 70g of ethanol was taken, and 22. 5g of hydroxypropyl cellulose (Nisso HPC (SL grade), Nippon Soda Co., Ltd.) was added thereto slowly with stirring, and was then dissolved completely by stirring for about 30 minutes. Next, 7.5g of famotidine, which is a gastric ulcer drug, was added, and stirring was carried out for about a further 5 minutes.

**[Table 2]**

| Liquid B | |
|---|---|
| Famotidine | 7.5g |
| Hydroxypropyl cellulose | 22.5g |
| Ethanol | 70.0g |

The liquid A was degassed, and then this solution was applied onto a surface of a 38µm-thick polyethylene terephthalate film (hereinafter referred to as 'PET film') onto which a silicone resin releasing agent had been applied, and drying was carried out for about 10 minutes at 80°C, thus forming a water-swellable gel-forming layer of thickness about 50µm. Next, the liquid B was degassed, and then this solution was applied onto the water-swellable gel-forming layer, and drying was carried out for about 5 minutes at 80°C, thus forming a drug-containing layer of thickness about 70µm. The liquid A was then further applied onto the drug-containing layer, and drying was carried out for about 10 minutes at 80°C, thus forming a water-swellable gel-forming layer of thickness about 50µm. Inthisway, an orally administered agent/supporting substrate complex in which an orally administered agent (film-shaped preparation comprising three layers, i.e. a water-swellable gel-forming layer, a drug-containing layer, and a water-swellable gel-forming layer) is supported on the above-mentioned PET film as a supporting substrate was manufactured (see Fig. 8). The orally administered agent was punched out to a diameter of 30mm, and in the undermentioned tests the orally administered agent was used after being peeled off from the PET film.

### (2) Manufacture of intestine-soluble preparation and intestine-soluble preparation/supporting substrate complex

The liquid A of the composition shown in Table 1 above was prepared as described above for forming water-swellable gel-forming layers.

A liquid C of the composition shown in Table 3 below was prepared for forming a drug-containing layer. That is, 35g of each of acetone and ethanol were taken, and 22.5g of hydroxypropyl methyl cellulose phthalate (Kanto Kagaku) was added thereto slowly with stirring, and was then dissolved completely by stirring for about 30 minutes. Next, 7.5g of the above-mentioned famotidine was added, and stirring was carried out for about a further 5 minutes.

**[Table 3]**

| Liquid C | |
|---|---|
| Famotidine | 7.5g |
| Hydroxypropyl methyl cellulose phthalate | 22.5g |
| Acetone | 35.0g |
| Ethanol | 35.0g |

The liquid A was degassed, and then this solution was applied onto a surface of a 38µm-thick polyethylene terephthalate film (hereinafter referred to as 'PET film') onto which a silicone resin releasing agent had been applied, and drying was carried out for about 10 minutes at 80°C, thus forming a water-swellable gel-forming layer of thickness about 50µm. Next, the liquid C was degassed, and then this solution was applied onto the water-swellable gel-forming layer, and drying was carried out for about 5 minutes at 70°C, thus forming a drug-containing layer of thickness about 70µm. The liquid A was then further applied onto the drug-containing layer, and drying was carried out for about 10 minutes at 80°C, thus forming a water-swellable gel-forming layer of thickness about 50µm. In this way, an orally administered agent/supporting substrate complex in which an orally administered agent (film-shaped preparation comprising three layers, i.e. a water-swellable gel-forming layer, a drug-containing layer, and a water-swellable gel-forming layer) is supported on the above-mentioned PET film as a supporting substrate was manufactured (see Fig. 8). The orally administered agent was punched out to a diameter of 30mm, and in the undermentioned tests the orally administered agent was used after being peeled off from the PET film.

### (3) Manufacture of orally administered agent/supporting substrate complex suitable for oral administration

In (1) and (2) above, a PET film of length 100mm, width 25mm and thickness 38µm was used as the supporting substrate, thus manufacturing an orally administered agent/supporting substrate complex as in Fig. 10. This supporting substrate has a part that can be grasped in the hand (gripping part) (length 30mm) and a part that can be inserted into the mouth of a patient or the like (mouth-inserting part) (length 70mm), and the orally administered agent is provided on the mouth-inserting part.

### [Test example 1]

### Evaluation tests into ease and safety of taking agent, and masking of taste of drug

Ten randomly sampled test subjects 10 were asked to take without water the orally administered agents manufactured in manufacturing examples 1 (stomach-soluble preparation and intestine-soluble preparation), and the ease of taking the agent and the drug taste masking ability were evaluated in accordance with the 5-level evaluation criteria described below. Moreover, at the same time evaluation was also carried out into whether or not the agent got stuck in the throat, airway or esophagus when taken (i.e. the safety of taking the agent)

### [Evaluation criteria for ease of taking agent]

1: Doesn't swell and gelate, cannot be taken without water.
2: Swells and gelates slightly, but cannot be taken without water.
3: Swells and gelates, but would like to take with water if possible.
4: Swells and gelates slowly, and can be taken without water.
5: Swells and gelates rapidly, and can be taken without water.

### [Evaluation criteria for masking ability]

1: Taste of drug spreads through mouth as soon as put into mouth, problems with taking.
2: No taste immediately after putting into mouth, but taste appears before swallowing, and hence problems with taking.
3: Taste of drug sensedwhile in mouth, but to extent that no problems with taking.
4: Taste of drug hardly sensed.
5: No taste of drug whatsoever.

The results for the stomach-soluble preparation are shown in Table 4 below, and the results for the intestine-soluble preparation are shown in Table 5 below.

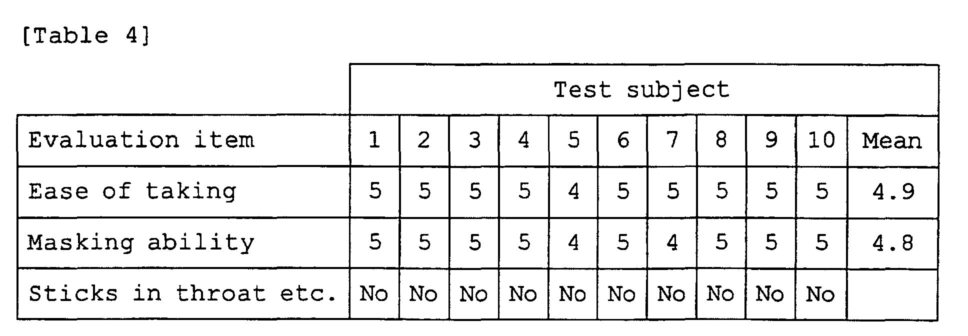

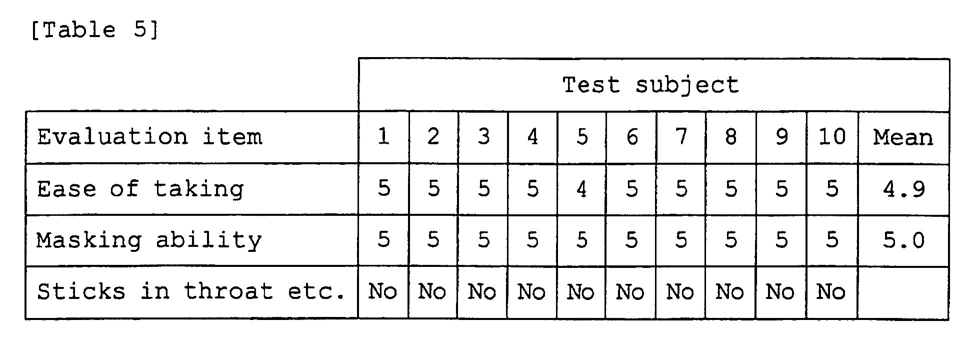

As shown in Tables 4 and 5, the orally administered agents (stomach-soluble preparation and intestine-soluble preparation) manufactured in manufacturing examples 1 were excellent in terms of ease and safety of taking the agent, and masking of the taste of the drug.

### [Test example 2]

### Evaluation tests into film strength

An orally administered agent that was a film-shaped preparation (stomach-soluble preparation or intestine-soluble preparation) was manufactured as in manufacturing example 1 (1) or (2), and the film strength for the case that the drug content in the drug-containing layer was changed was measured in accordance with a JIS tensile strength test (JIS Z0237). The composition and thickness of the water-swellable gel-forming layer in the film-shaped preparation were made to be the same conditions as in manufacturing example 1 (1) or (2), but the number of water-swellable gel-forming layers was made to be one. Moreover, the drug content in the drug-containing layer was changed between 0.1wt%, 10wt%, 25wt%, 50wt%, and 80wt%.

The results of the film strength (kg/cm²) for the stomach-soluble preparation and the intestine-soluble preparation are shown in Table 6 below.

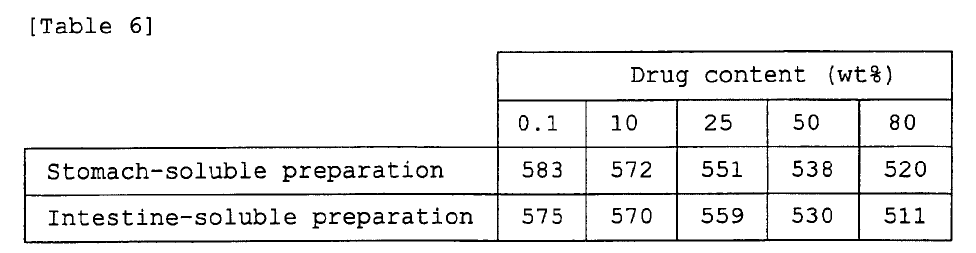

As shown in Table 6, in the film-shaped preparations manufactured in manufacturing example 1, a sufficient film strength could be maintained even when the drug content (wt%) in the drug-containing layer was changed over a broad range of 0.1 to 80wt%.

### [Test example 3]

### Evaluation tests into ease of administration using orally administered agent/supporting substrate complex, and ease of checking whether agent has been taken

An orally administered agent supported in an orally administered agent/supporting substrate complex manufactured as in manufacturing example 1 (3) was administered without water to ten randomly sampled test subjects who were lying down, and the ease of administration and the ease of checking whether the agent has been taken were evaluated in accordance with the following evaluation criteria.

### [Evaluation criteria for ease of administration]

1: Hand gets soiled, administration difficult.
2: Sometimes get hand soiled, but administration possible.
3: Don't get hand soiled at all, can administer easily.

### [Evaluation criteria for ease of checking whether agent has been taken]

1: Cannot check at all.
2: Checking difficult, but can check whether or not taking of agent has been completed.
3: Can check at a glance whether or not taking of agent has been completed.

The results of the evaluation tests are shown in Table 7 below.

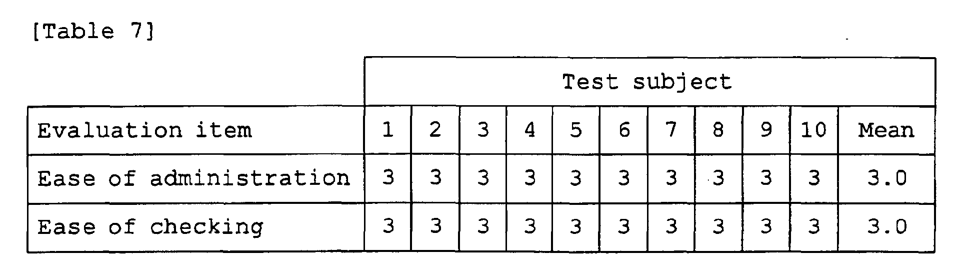

As shown in Table 7, the orally administered agent supported in an orally administered agent/supporting substrate complex gelated within 5 minutes inside the mouth of a test subject, and hence the orally administered agent could easily be administered even if the test subject was lying down. Moreover, by taking the supporting substrate on which the orally administered agent was supported out of the mouth of the test subject, it could easily be checked whether or not taking of the agent had been completed. Moreover, during the sequence of operations from administration to checking whether the agent had been taken, there was no soiling whatsoever of the hand or fingers by the test subject's saliva or the like. Moreover, in the case that the amount of secretion of saliva is low as with an elderly person or the like, one would think that it is necessary to dip the film-shaped preparation in water before administration, but by using the orally administered agent/supporting substrate complex, this dipping in water could also be carried out easily.

### [Test example 4]

### Study into mixing proportions of water-swellable gel-forming agent and film-forming agent in water-swellable gel-forming layers

The mixing proportions (wt%) of the water-swellable gel-forming agent and the film-forming agent in the water-swellable gel-forming layers were changed, and the solubility and the swellability of the film-shaped preparations when actually in the mouth were evaluated using the senses. The film-shaped preparations were manufactured in accordance with manufacturing example 1 (1) ; the composition of the drug-containing layer was set to be as in manufacturing example 1 (1), and the composition of the water-swellable gel-forming layers was set as in Table 8 below (the units are wt%).

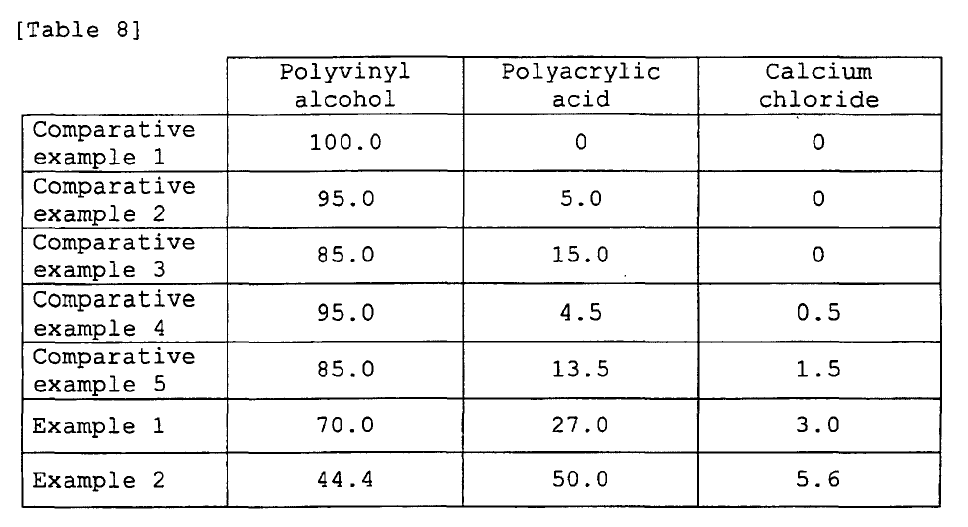

The results of the test are shown in Table 9 below.

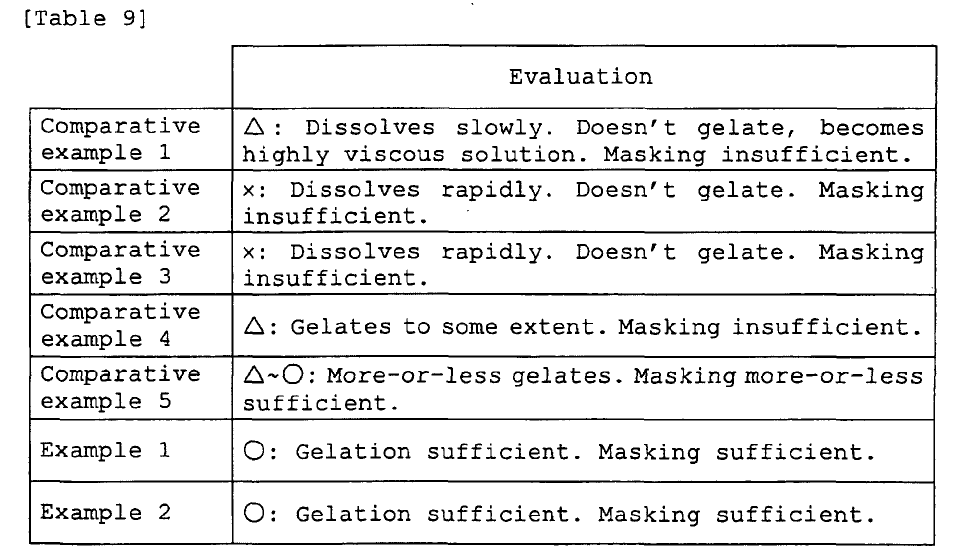

### INDUSTRIAL APPLICABILITY

According to the present invention, an orally administered agent according to which the ease and safety of taking the agent are improved, a film-shaped orally administered agent that can contain a broad range of types of drugs, and an orally administered agent according to which a drop in compliance of taking a drug due to the taste (e.g. bitterness, astringency) or smell of the drug can be prevented, are provided. Moreover, according to the present invention, an orally administered agent/supporting substrate complex according to which handling of an orally administered agent (e.g. carrying, storage etc. of the orally administered agent) can be made easy, an orally administered agent/supporting substrate complex according to which an orally administered agent can be administered easily, and an orally administered agent/supporting substrate complex according to which it can easily be checked whether or not an orally administered agent has been taken, are provided.

## Claims

1. An orally administered agent comprising a drug-containing layer and a water-swellable gel-forming layer, wherein said water-swellable gel-forming layer is provided as an outermost layer of said orally administered agent.

2. The orally administered agent according to claim 1, wherein said orally administered agent is a film-shaped preparation.

3. The orally administered agent according to claim 2, wherein said water-swellable gel-forming layer contains a water-swellable gel-forming agent and a film-forming agent.

4. The orally administered agent according to claim 3, wherein said water-swellable gel-forming agent is a cross-linked carboxyvinyl polymer, and said film-forming agent is a polyvinyl alcohol.

5. The orally administered agent according to claim 4, wherein said cross-linked carboxyvinyl polymer is a carboxyvinyl polymer cross-linked by a polyvalent metal compound.

6. The orally administered agent according to any of claims 3 through 5, wherein the content of said water-swellable gel-forming agent in said water-swellable gel-forming layer is 15 to 70wt%, and the content of said film-forming agent in said water-swellable gel-forming layer is 30 to 85wt%.

7. The orally administered agent according to any of claims 1 through 6, wherein said water-swellable gel-forming layer can mask the taste and/or smell of a drug contained in said drug-containing layer.

8. The orally administered agent according to any of claims 1 through 7, wherein said drug-containing layer contains an edible polymer as a base.

9. The orally administered agent according to claim 8, wherein said edible polymer is cellulose and/or a cellulose derivative.

10. The orally administered agent according to claim 8 or 9, wherein the content of said edible polymer in said drug-containing layer is at least 20wt%.

11. An orally administered agent/supporting substrate complex comprising the orally administered agent according to any of claims 1 through 10, and a supporting substrate that supports the orally administered agent, wherein said orally administered agent is provided on said supporting substrate either directly or via an intermediate layer.

12. The orally administered agent/supporting substrate complex according to claim 11, wherein said supporting substrate has a gripping part and a mouth-inserting part, and said orally administered agent is provided on said mouth-inserting part.
